# EUROPEAN PATENT SPECIFICATION

(11) **EP 3 116 413 B1**
(45) Date of publication and mention of the grant of the patent: **06.06.2018**
(21) Application number: 15712498.3
(22) Date of filing: 13.03.2015
(51) Int. Cl.: A61B 17/115, A61B 17/122

(54) **APPARATUS FOR CLIPPING TISSUE**
VORRICHTUNG ZUM SCHNEIDEN VON GEWEBE
APPAREIL POUR ATTACHER UN TISSU

(30) Priority: 14.03.2014 US 201461953312 P
(43) Date of publication of application: 18.01.2017
(73) Proprietor: Boston Scientific Scimed, Inc., Maple Grove, MN 55311-1566 (US)
(72) Inventor: POWERS, Michael, Pepperell, Massachusetts 01463 (US); SMITH, Paul, Smithfield, Rhode Island 02917 (US); RAYBIN, Samuel, Marlborough, Massachusetts 01752 (US); SCHWARTZ, Bernard B., East Dorset, VT 05253 (US); GOLDEN, John B., Norton, Massachusetts 02766 (US); SOUN, Naroun, Lawrence, Massachusetts 01843 (US); BEAN, Jeff V., Fitchburg, Massachusetts 01420 (US)
(74) Representative: Vossius & Partner Patentanwälte Rechtsanwälte mbB
(86) International application number: PCT/US2015/020438
(87) International publication number: WO 2015/138890

(56) References cited:
- EP-A1- 1 749 506
- US-A- 5 078 731
- US-A1- 2007 219 571
- US-A1- 2013 231 686

## Description

### FIELD OF THE DISCLOSURE

Embodiments of the present disclosure relate to devices for treating body tissues, and more particularly for clipping and/or compressing body tissues.

### BACKGROUND

Surgical clips are used during a variety of medical procedures to, for example, hold or clamp tissues together, squeeze blood vessels or other bodily lumens to reduce or prevent bleeding, etc. Clips may be introduced into a patient's body using various approaches, such as a subcutaneous approach, a minimally invasive approach, etc. In a subcutaneous approach, a patient's skin is penetrated to obtain entrance to a target site within the body to deploy a clip over target tissue, (e.g., to close a wound or surgical opening). In one approach, a clip is maneuvered to a target site within the body via an endoscope or other introducer inserted through a body lumen accessed, for example by a natural anatomical opening such as the mouth.

Certain treatments involve the deployment of multiple clips within the body. Many related art systems deliver only one clip at a time. If a second clip is required, the system or delivery instrument must be removed from the body to receive another clip or an entirely new clipping device. This process must be repeated until the desired number of clips has been deployed. As a result, the deployment of multiple clips may be complicated, time consuming, and expensive.

The following implementations may reduce the time and effort required to deploy multiple clips, simplifying the system, and/or performing this process more economically.

US 2007/0219571 A1 discloses endoscopic plicators passed transorally into the stomach and used to plicate stomach tissue by engaging tissue from inside of the stomach and drawing it inwardly.

### SUMMARY

The present invention is defined in claim 1. The first clip may further comprise a first locking member coupled to the first jaw and extending from the first jaw toward the second jaw, the first locking member being constructed so that, when the first and second jaws are moved toward one another into the second configuration, the first locking member lockingly engages the first jaw member.

In an embodiment, the first locking member may be formed as a rod extending from the first jaw toward the second jaw.

In an embodiment, a free end of the rod of the first locking member may include a locking arm extending away from the rod at an angle offset from a longitudinal axis of the rod to engage the second jaw.

In an embodiment, a proximal surface of the free end of the locking arm may be angled relative to a surface of the second jaw so that, as the free end of the rod moves proximally into contact with the second jaw, the locking arm deflects until the locking arm passes over the second jaw and locks against a proximal side thereof.

In an embodiment, the first jaw may extend from a first end including a first body engaging the collapsible member to a second end including a first tissue gripping tip.

In an embodiment, the second jaw may extend from a first end including a second body engaging the collapsible member to a second end including a second tissue gripping tip.

In an embodiment, the second end of the second jaw may include a third tissue gripping tip.

In an embodiment, the control wire may include an enlarged distal end engaging distal side of the opening in the first jaw so that proximal movement of the control wire relative to the first jaw draws the first jaw proximally relative to the second jaw, the enlarged end being configured to be movable to a second configuration to permit separation thereof from the first jaw to release the first clip.

In an embodiment, the enlarged distal end of the control wire may engage the opening in the first jaw with a pressure fit.

In an embodiment, the opening in the second jaw may include a rod receiving space and the rod may be lockingly received within the second channel in the second contracted configuration.

In an embodiment, the second and third tissue gripping tips may be separated from one another by an opening, the opening receiving at least a portion of the first jaw therein in the second contracted configuration, the opening includes an angled wall configured to lockingly engage the locking arm of the first locking member.

In an embodiment, the collapsible member may include first and second tabs received within corresponding slots formed in the first and second jaw.

In an embodiment, the device may further comprise a collapsible body portion connecting the first and second tabs, including first and second portions connected to one another at a joint, wherein in the first expanded configuration, the first and second portions are moved apart to enclose a first angle therebetween and wherein, in the second contracted configuration, the first and second arms enclose a second angle smaller than the first angle.

In an embodiment, the second angle may be 0.

In an embodiment, an outer profile of the first clip may be reduced when housed within the flexible insertion member, the outer profile expanding to a biased configuration when the first clip is advanced out of the flexible insertion member.

In an embodiment, the device may further comprise a second clip including third and fourth jaws, each of the third and fourth jaws including an opening through which the control wire passes and a second clip collapsible member coupled between the third and fourth jaws, the second clip collapsible member being biased to urge the third and fourth jaws away from one another along an axis of the control wire to a first expanded configuration, the third and fourth jaws being movable against the bias of the second clip collapsible member via the control wire to a second contracted configuration in which the third and fourth jaws are moved along the axis of the control wire toward one another to lockingly engage tissue received therebetween.

A method for causing hemostasis includes advancing a clip through a flexible insertion member to a target tissue site. The clip includes a first jaw and a second jaw coupled to one another by a collapsible member. Each of the first and second jaws includes an opening through which a control wire passes. The collapsible member is biased to urge the first and second jaws away from one another along an axis of the control wire. The method further includes actuating the control wire to move the first and second jaws out of the insertion member and then drawing tissue into a space between the first and second jaws. The method further includes withdrawing the control wire proximally a first distance to move the first jaw along an axis of the control wire proximally toward the second jaw until a locking element couples the first and second jaws to one another to lockingly engage tissue received therebetween. The method further includes withdrawing the control wire proximally by a second distance to disengage the control wire from the first and second jaws to release the clip from the control wire.

It is to be understood that both the foregoing general description and the following detailed description are exemplary and explanatory only and are not restrictive of the disclosure, as claimed.

### BRIEF DESCRIPTION OF THE DRAWINGS

The accompanying drawings, which are incorporated in and constitute a part of this specification, illustrate exemplary embodiments of the present disclosure and together with the description, serve to explain the principles of the disclosure.
FIG. 1 is a schematic side view of an exemplary embodiment of a clipping device.
FIG. 2 is a schematic side view of a clipping device according to a first alternate embodiment of the disclosure.
FIG. 3 is a schematic side view of a clipping device according to a second alternate embodiment of the disclosure.
FIG. 4 is a schematic side view of a clipping device according to a third alternate embodiment of the disclosure.
FIG. 5 is a schematic side view of a clipping device according to a fourth alternate embodiment of the disclosure.
FIG. 6 is a schematic side view of a clipping device according to a fifth alternate embodiment of the disclosure.
FIG. 7 is a schematic side view of a clipping device according to a sixth alternate embodiment of the disclosure.
FIG. 8 is a perspective view of a clipping device according to a seventh alternate embodiment of the present disclosure.
FIG. 9 is a perspective view of a clip of the device of FIG. 8, in an open configuration.
FIG. 10 is a perspective view of the clip of the device of FIG. 8, in a partially closed configuration.
FIG. 11 is a perspective view of the clip of the device of FIG. 8, locked in a closed configuration.
FIG. 12 is an enlarged perspective view showing contact between two adjacent clips of the device of FIG. 8.
FIG. 13 is a schematic side view of the device of FIG. 8.
FIG. 14 is an enlarged schematic perspective view of a portion of the clip of the device of FIG. 8.
FIG. 15 is a longitudinal cross-sectional side view of the portion of the clip of FIG. 14.
FIG. 16 is a longitudinal cross-sectional side view of a portion of a clip according to an alternate embodiment of the present disclosure.
FIG. 17 is a schematic perspective view of the portion of the clip of FIG. 16.
FIG. 18 is a perspective view of a clip according to an eighth alternate embodiment of the present disclosure.
FIG. 19 is a schematic side view of the clip of FIG. 18.
FIG. 20 is a perspective view of a clip according to a ninth alternate embodiment of the present disclosure.
FIG. 21 is a partially transparent perspective view of the clip of FIG. 21, in a closed configuration.
FIG. 22 is a perspective view of a clip according to a tenth alternate embodiment of the present disclosure.
FIG. 23 is a perspective view of a clip according to an eleventh alternate embodiment of the present disclosure.
FIG. 24 is a perspective view of a clip according to a twelfth alternate embodiment of the present disclosure.
FIG. 25 is an enlarged perspective view of a portion of the clip of FIG. 24.
FIG. 26 is a perspective view of a clip according to a thirteenth alternate embodiment of the present disclosure, in an open configuration.
FIG. 27 is a perspective view of the clip of FIG. 26, in a closed configuration.
FIG. 28 is a perspective view of a clip according to a fourteenth alternate embodiment of the present disclosure.
FIG. 29 is a perspective view of the clip of FIG. 28, in a closed configuration.
FIG. 30 is a perspective view of a clip according to a fifteenth alternate embodiment of the present disclosure.
FIG. 31 is an enlarged cross-sectional view of a portion of the clip of FIG. 30.

### DESCRIPTION OF THE EMBODIMENTS

Reference will now be made in detail to embodiments of the present disclosure, examples of which are illustrated in the accompanying drawings. Wherever possible, the same reference numbers will be used throughout the drawings to refer to the same or like parts.

Embodiments of the present disclosure relate to devices for clipping tissue. The clipping device includes a flexible insertion member that can be inserted into a body along a tortuous path (e.g., through a natural body lumen accessed via a natural body opening, such as the mouth). The flexible insertion member is configured to receive one or more clips therethrough, the clips being configured to engage a portion of body tissue within the body. The exemplary clip includes first and second jaws, each including an opening through which a control wire passes. A collapsible member coupled between the first and second jaws is biased to urge the first and second jaws away from one another along an axis of the control wire to a first expanded configuration. The first and second jaws are moved toward one another against the bias of the collapsible member via the control wire to a second retracted configuration to lockingly engage tissue received therebetween.

Those skilled in the art will recognize that the present disclosure may be manifested in a variety of forms other than the specific embodiments described and contemplated herein. Accordingly, departure in form and detail may be made without departing from the scope of the invention as defined by the appended claims.

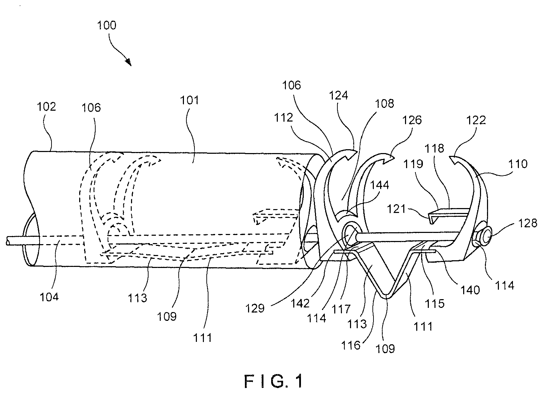

FIG. 1 is a schematic side view of an exemplary embodiment of a clipping device 100. The clipping device 100 includes a flexible insertion member 102 which, in this embodiment, is sized and shaped for insertion through a working channel of an insertion device (e.g., an endoscope) (not shown). The insertion member 102 defines a lumen 101 extending along an axial length thereof between a proximal end (not shown) and a distal end 103. The insertion member 102 is sized so that, when a distal end 103 thereof is inserted to a target site within the body, a proximal end thereof (not shown) remains outside the body accessible to a user. Typically, the insertion member 102 is introduced into the patient's body through a naturally occurring anatomical opening, such as the mouth.

The insertion member 102 in this exemplary embodiment is formed of a flexible biocompatible material. For example, the insertion member 102 may be formed of a polymer such as polytetrafluoroethylene (PTFE), polyurethane, and so forth. Other suitable materials such as composites, metals, and so forth may also be used without deviating from the scope of the disclosure.

The clipping device 100 further includes one or more clips 106 housed within the lumen 101. Dimensions of the lumen 101 are selected to facilitate uninterrupted advancement of the clips 106 therethrough and out of a distal opening of the insertion member 102. To this end, the diameter of the lumen 101 is selected to be equal to or slightly larger than a profile of the clip 106.

The clip 106 includes a first jaw 110 and a second jaw 112 coupled to each other via a clip collapsible member 116. The collapsible member 116 includes first and second portions 111 and 113 connected to one another at a joint 109 forming an angle α therebetween. In some embodiments, the collapsible member 116 may be formed of a flexible, resilient material. The collapsible member 116 is biased toward an open position in which the angle α is increased may be moved when subjected to a compressive force to a closed, tissue-receiving position in which the angle α is reduced to bring the first and second jaws 110, 112 toward one another, as will be described in greater detail with respect to the exemplary method below. The first jaw 110 includes a first tissue gripping tip 122. The second jaw 112 includes two tissue gripping tips 124, 126 which form a V-shaped configuration defining an opening 108 therebetween. The second and third tips 124, 126 are configured to permit insertion of the first tip 122 therebetween when the clip 106 is moved to a locked configuration, as will be described in greater detail later on with respect to the exemplary method. The first and second jaws 110, 112 also include openings 114 extending therethrough sized to permit slidable insertion of a control wire 104 therethrough. A diameter of the opening 114 is selected to be equal to or slightly greater than a diameter of an increased diameter portion 128, 129 provided on the control wire 104. As will be described in greater detail later on, the increased diameter portion 128, 129 is adapted to be pulled through the opening 114 upon application of a predetermined threshold pressure thereto. It is noted that although the embodiments depicted herein include two increased diameter portions 128, 129, an alternate embodiment may include only one increased diameter portion 128 without changing the mode of operation of the device 100.

As indicated above, the collapsible member 116 is biased toward an open position, in which the distal end of the portion 111 is separated from the proximal end of the portion 113 while their joined ends form an angle α therebetween. In the open position, the angle α is selected to be sufficiently close to 180 degrees so that the clip 106 may pass through the lumen 101. In the open position, the angle α between the two portions 111 and 113 can be as great as 180 degrees such that the two portions 111 and 113 are substantially co-linear substantially parallel to a longitudinal axis of the control wire 104, although a smaller angle may be used without deviating from the scope of the disclosure. For example, any angle smaller than 180 degrees may be used as long as an outer profile of the collapsible member 116 is small enough to permit insertion of the assembled clip through the lumen 101.

The collapsible member 116 is movable to a closed position by pulling the control wire 104 proximally to cause the first jaw 110 to move proximally toward the second jaw 112, as will be described in greater detail with respect to the exemplary method below. A hypotube (not shown) is positioned proximally of a proximalmost one of the clips 106 to prevent the clips 106 from being withdrawn proximally as the control wire 104 is retracted. Rather, the hypotube (not shown) remains in position against a proximal end of the proximalmost clip 106, countering the proximally directed force from the control wire 104 and causing the collapsible member 116 to move to the closed position. The hypotube (not shown) is formed as an elongated hollow tube housed within the lumen 101 and having an opening extending therethrough, the opening being sized to permit the control wire 104 to be slidably received therethrough and prevent the clip 106 from being drawn thereinto. In the closed configuration, the angle α formed between the two portions 111, 113 is reduced relative to the open configuration. In one embodiment, the angle α is 0 degrees in the closed position such that the two portions 111 and 113 are pressed against one another. The collapsible member 116 further includes a first tab 115 coupling the collapsible member 116 to the first jaw 110 and a second tab 117 coupling the collapsible member 116 to the second jaw 112. The first and second jaws 110 and 112 include first and second slots 140, 142 configured to receive the first and second tabs 115 and 117, therein. The collapsible member 116 may be permanently attached to the first and second jaws 110, 112 using an adhesive or any other attachment means known in the art.

Generally, the clips 106 may remain in the open, expanded configuration when housed within the lumen 101 of the insertion member 102. In the expanded configuration, the jaws 110, 112 of clips 106, are separated from one another by a distance substantially equal to the length of the collapsible member 116.

The clip 106 further includes a locking member 118 extending from an inner surface of the first jaw 110 toward the second jaw 112. The locking member 118 in this embodiment is a rod 119 extending proximally from a first end coupled to the first jaw 110 to a free end with a locking arm 121 extending away therefrom at an angle. The rod 119 in this embodiment is substantially parallel to a longitudinal axis of the control wire 104 while the locking arm 121 extends away from the free end as an extension angled downward relative to the rod 119 and forming an acute angle therewith. In an operative configuration, the first jaw 110 is drawn toward the second jaw 112 and, as the locking member 118 passes through the opening 108, the locking arm 121 engages a wall 144 at the opening 108. The wall 108 is angled so that, as the locking member 118 slides thereover, the locking member 118 is deflected upward. Once the locking arm 121 has passed proximally beyond the wall 144, a biasing force causes the locking member 118 to deflect downward to its original configuration. In this position, the locking arm 121 lockingly engages a proximal wall of the second jaw 112 locking the first and second jaws 110, 112 together in the locked configuration. As would be understood by those skilled in the art, the locking member 118 is formed of a material that is substantially flexible so that the locking member 118 is capable of deflecting over the wall 108 without fracturing. Alternatively, the locking member 118 may include a predetermined structure, such as a bend that can be deflected upon meeting the second jaw 112, such that the two jaws 110 and 112 can be engaged in the locked state.

In accordance with an exemplary method, the insertion device 102 is loaded with a predetermined number of clips 106 corresponding to the requirements of a particular procedure. The insertion device 102 is then advanced to a target site in the body. Once the target location is reached, the control wire 104 and hypotube (not shown) are advanced distally to effect a corresponding distal advancement of the clips 106 until a distal-most one of the clips 106 exits the lumen 101 facing the target tissue site. Specifically, the control wire 104 includes first and second enlarged portions 128, 129 provided thereon, the enlarged portions having substantially spherical shapes although other shapes are envisioned within the scope of the disclosure. As the control wire 104 is advanced distally, engagement of the second enlarged portion 129 with the opening 114 causes the second jaw 112 to move distally. In another embodiment, the hypotube (not shown) may be advanced distally to cause a corresponding distal movement of the clips 106, wherein distal advancement of the hypotube causes a corresponding distal advancement of the control wire 104. As the distal-most clip 106 exits the lumen 101, the collapsible member 116 remains in the open configuration so that the first and second portions 111, 113 enclose an angle of approximately 180 degrees. Once the clip 106 has been moved to a desired position (e.g., under visual guidance), target tissue is drawn into the space between the first and second jaws 110, 112 using graspers or any mechanism known in the art. The control wire 104 is then retracted proximally to draw the first enlarged portion 128 proximally to exert a proximally directed force on the first jaw 110, moving the first jaw 110 proximally toward the second jaw 112. As the first jaw 110 moves proximally, the first and second portions 111, 113 bend about the joint 109 so that the angle α begins to approach 0 degrees. Continued retraction of the control wire 104 causes the locking member 118 to penetrate the target tissue and advance toward the opening 108 until the locking arm 121 extends proximally past the wall 144 and locks the first and second jaws 110, 112 in a locked configuration with the target tissue lockingly received between the jaws 110, 112. In this position, contact between the first and second portions 111, 113 of the collapsible member 116 prevents the first jaw 110 from moving further proximally toward the second jaw 112. Furthermore, the increased profile of the bent collapsible member 116 prevents the clip 106 from being retracted back into the insertion member 102. As the control wire 104 is retracted further proximally, a force exerted by the first enlarged portion 128 against the material surrounding opening 114 increases until a threshold level is exceeded. At this point, the first enlarged portion 128 is forced through the opening 114 and drawn proximally away from the first jaw 110. Continued proximal withdrawal of the control wire 104 now exerts an increasing force against the material surrounding the opening 114 of the second jaw 112 to separate the clip 106 from the control wire 104. This process may then be repeated as necessary to deploy any number of clips 106 within the body using the same insertion member 102 and control wire 104. Although the illustrated embodiment shows only two clips 106, it should be understood that any number of clips 106 can be employed including, but not limited to, three, four, five, six, and so forth. In fact, some embodiments only include a single clip 106, even though the above structure lends itself to multiple clips 106.

Since the clipping device 100 is capable of passing multiple clips 106 through the insertion member 102, the clipping device 100 may be suited for treating larger tissue wounds. For example, a distal-most clip 106 may be deployed at a first end of the tissue wound, the first and second jaws 110, 112 grasping tissue along opposing edges of the tissue wound in the open configuration. The first clip 106 may then be moved to the closed configuration to draw the tissue along opposing edges toward one another. Subsequent clips 106 may be deployed along a length of the tissue wound in a similar manner, until the entire tissue wound is closed.

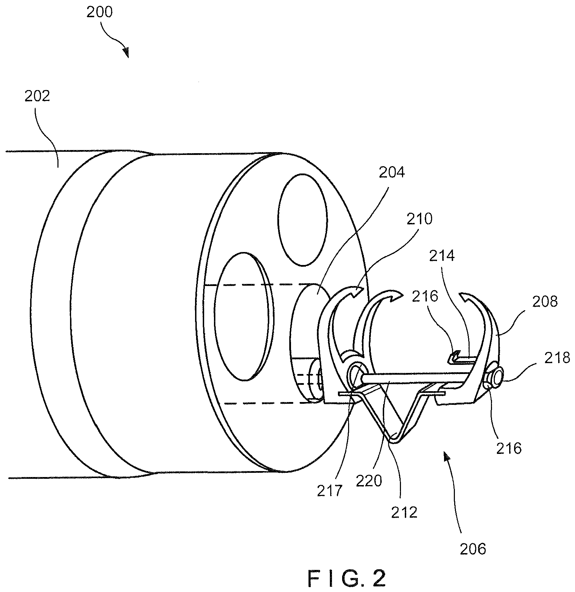

FIG. 2 illustrates another exemplary clipping device 200. As shown, the clipping device 200 includes a clip 206 that is inserted into the body using an endoscope 202. However, any other suitable insertion member, such as an introduction sheath, cannula, and so forth may also be employed to introduce the clip 206 into the body.

The endoscope 202 may receive one or more clips, such as the clip 206 in its working channel 204. Although not referenced, the endoscope may have multiple working channels each having a similar form and function as that of the working channel 204. In the illustrated embodiment, the clip 206 includes a first jaw 208 and a second jaw 210 coupled to one another via a collapsible member 212. Each of the first and second jaws 208 and 210 defines an opening 216, 217 through which a control wire 220 passes. The control wire 220 includes an enlarged distal end 218 that prevents any inadvertent dislodgement of the clip 206. Whereas the locking arm 121 of the clip 106 extended away from the was bent away from the locking member 118 toward the collapsible member 116, a locking member 214 of the clip 206 includes a locking arm 216 bent toward tips 122, 124, 126. When moved to the locking configuration, the locking member 214 enters the opening 217 and bends away from the edge of the opening until the end thereof passes this edge. That is, the opening 217 is sized to permit insertion of the locking member 214 thereinto without interfering with slidable movement of the control wire 220. At this point, the locking member 214 straightens to its original shape and the locking arm 216 locks the first jaw 208 to the second jaw 210.

It should be understood that the components of the clip 206, as discussed above, are similar in form and function to the components of clip 106 of FIG. 1. However, for the purpose of clarity and brevity, the components of the clip 206 are labeled differently.

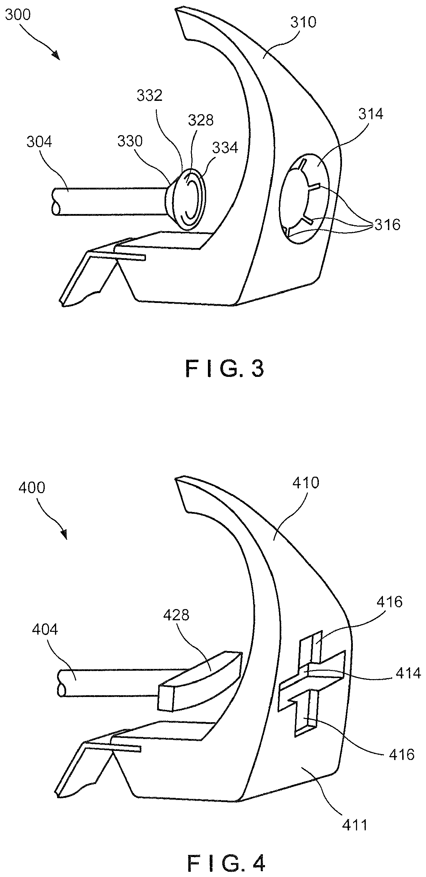

FIG. 3 depicts a device 300 according to another embodiment of the disclosure. The device 300 is substantially similar to the device 100, wherein like elements have been referenced with like reference numerals, except as noted below. Specifically, whereas the control wire 104 includes an enlarged substantially spherical abutment 128 at a distal end thereof, a control wire 304 of the device 300 includes a disc-shaped abutment 328 formed to engage a circular opening 314 in a first jaw 310. The disc-shaped abutment 328 includes a first linearly tapered wall 330 extending from an outer wall of the control wire 304 to a maximum diameter region 332. The abutment 328 also includes a curved tapered wall 334 extending to an apex at a distalmost end thereof. The opening 314 is formed as a circular cutout extending through the first jaw 310 and including a plurality of slots 316 extending radially thereoutof through the first jaw 310. A curved wall 318 leads into the opening 314 from a distal end thereof, a curvature of the curved wall 318 being selected to seat the abutment 328 flush therein in an operative configuration.

In an operative configuration, the abutment 328 is positioned distally of the opening 314 in the same manner disclosed above with respect to the device 100. When it is desired to bring the first jaw 310 proximally toward the second jaw 112, the control wire 304 is retracted proximally to cause a corresponding proximal retraction of the first jaw 310 until the locking member 118 (not depicted in FIG. 3) lockingly engages the second jaw 112. Further proximal retraction of the control wire 304 causes the abutment 328 to exert a pressure on the opening 314. Once this pressure exceeds a predetermined threshold value, the slots 316 are radially compressed to temporarily increase a diameter of the opening 314 and permit the abutment 328 to be separated therefrom.

FIG. 4 depicts a device 400 according to another embodiment of the disclosure. The device 400 is substantially similar to the device 100, wherein like elements have been referenced with like reference numerals, except as noted below. Specifically, FIG. 4 depicts yet another attachment mechanism removably coupling a control wire 404 to a first jaw 410. The control wire 404 extends to an abutment 428 at a distal end thereof, the abutment 428 formed as a projection having a rectangular cross-section. The first jaw 410 includes an opening 414 extending therethrough. The opening 414 has a rectangular shape corresponding to the rectangular shape of the abutment 428 and 410 further comprises a recessed portion 416 extending thereinto from a distal surface 411 thereof by a distance less than a thickness of the first jaw 410. In one embodiment, a depth of the recessed portion 416 is substantially equivalent to a thickness of the abutment 428 so that the abutment 428 may be seated flush therein in an operative configuration. In another embodiment, the depth of the recessed portion 416 may be smaller than a thickness of the abutment 428. The recessed portion 416 is centered relative to the opening 414 and is oriented to extend perpendicular to the opening 414. It is noted that this orientation is exemplary only and that any other angular orientation of the recessed portion 416 relative to the opening 414 is envisioned within the scope of the disclosure. Similar to the opening 414, the recessed portion 416 has a rectangular cross-section to correspond to the rectangular shape of the abutment 428.

In an operative configuration, the abutment 428 is positioned distally of the opening 414 in the same manner disclosed above with respect to the device 100 such that the abutment is seated within the recessed portion 416. When it is desired to bring the first jaw 410 proximally toward the second jaw 112, the control wire 404 is retracted proximally to cause a corresponding proximal retraction of the first jaw 410 until the locking member 118 (not depicted in FIG. 4) lockingly engages the second jaw 112 as described in regard to the previous embodiments. One the first and second jaws 410, 112 are locked relative to one another, the control wire 404 is advanced distally a short distance to move the abutment 428 distally out of the recessed portion 416. The control wire 404 is then rotated 90° to align the abutment 428 with the opening 414. Once aligned, the control wire 404 is retracted proximally through the opening 141 to disengage the first jaw 410. The second jaw (not shown) of the device 400 may include an opening (not shown) sized to permit the abutment 428 to be retracted proximally therefrom., the opening (not shown) being aligned, for example, with the opening 414 so that the control wire 404 does not need to be rotated to disengage the second jaw. It is noted that although the abutment 428 is rectangular, any other polygonal shape is envisioned within the scope of the disclosure such that the abutment 428 is capable of engaging the first jaw 410 in the above-disclosed manner.

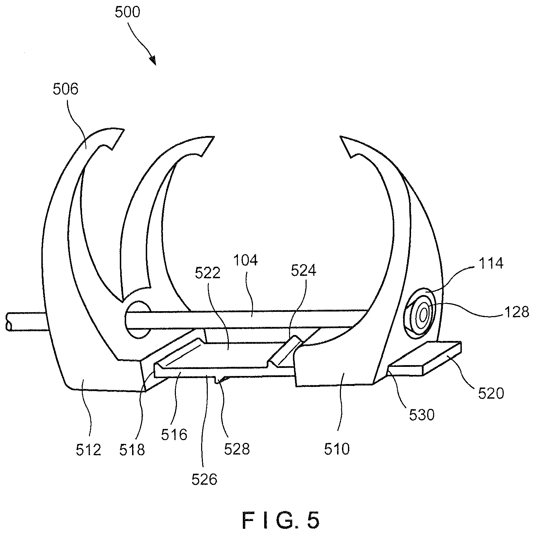

As shown in FIG. 5 a device 500 according to yet another embodiment of the disclosure, is substantially similar to the device 100 except as noted below. The exemplary embodiment of FIG. 5 omits the need for a locking member 118 on the first jaw and instead, employs a ratchet mechanism to lock a clip 506 in a closed configuration. The device 500 includes first and second jaws 510, 512 connected to one another by a ratchet member 516. The ratchet member 516 extends from a proximal end 518 permanently attached the second jaw 512 to a distal end 520. A first side wall 522 of the ratchet member 516 includes one or more first protrusions 524 extending therefrom and a second side wall 526 includes one or more second protrusions 528 extending therefrom. The first and second protrusions 524, 528 include angled walls formed to engage in a ratcheting manner an opening 530 extending through the first jaw 510, as will be described in greater detail hereinafter. The opening 530 has a rectangular cross-section conforming to the shape of the ratchet member 516 although other shapes are envisioned within the scope of the disclosure (e.g., square, oblong, etc.).

In an operative configuration, the device 500 is inserted through an endoscope to a target tissue site in the same manner disclosed above with respect to the device 100. As also disclosed above, a hypotube (not shown) or a control wire 104 is then advanced distally to move clip out of the endoscope. Once properly positioned, the control wire 104 or hypotube is retracted proximally to move the first jaw 510 proximally toward the second jaw 512. As the first jaw 510 slides proximally, the opening 530 ratchets over the first and second protrusions 524, 528 of the ratchet member 516. As those skilled in the art will understand, the ratchet mechanism 516 allows movement of the first jaw 510 in only one direction. Accordingly, once the first jaw 510 has moved proximally of any of the first and second protrusions 524, 528, the first jaw 510 is prevented from returning to a position distal thereof due to engagement of a wall (not shown) of the opening 530 with a wall (not shown) of the first and second protrusions 524, 528. The control wire 104 or hypotube is moved proximally until engagement of the first jaw 510 with the second jaw 512 prevents further proximal movement thereof. In this position, further proximal retraction of the control wire 504 or hypotube causes the abutment 128 to disengage from the opening 114 as disclosed in greater detail earlier.

In another embodiment, the device 500 may be formed so that the ratchet member 516 engages in a ratcheting manner an opening extending through the second jaw 512 instead of the first jaw 510.

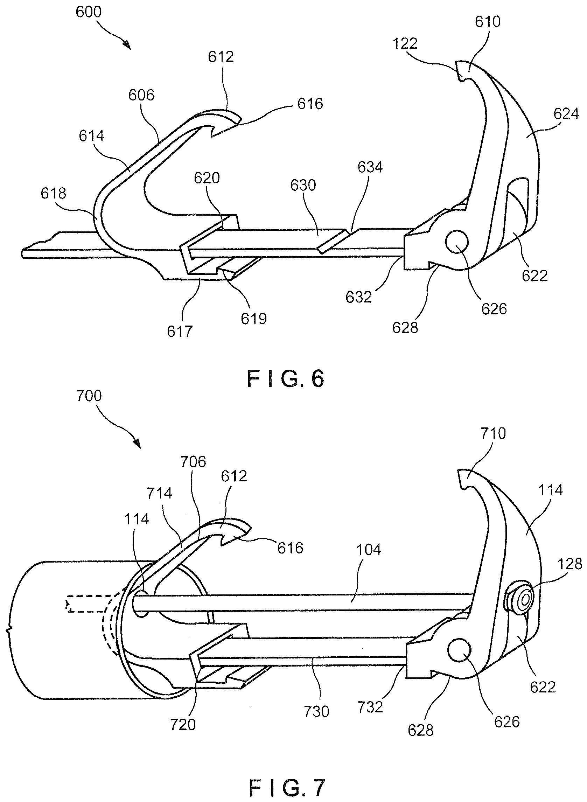

As shown in FIG. 6, a device 600 according to another embodiment of the disclosure is substantially similarly to the device 100 except as noted below. The device 600 includes first and second jaws 610, 612. The first jaw 610 includes a base portion 622 and a body portion 624 rotatably connected to one another by a pivot pin 626, with the body portion 624 extending to a first tissue gripping tip 122. A lower surface of the first jaw 610 includes a notch 628, whose purpose will be described in greater detail later on. The second jaw 612 extends from a body 614 to a second tissue gripping tip 616. The body 614 is formed of a material having elastic properties permitting the second jaw 612 to be folded about an apex 618 during insertion thereof through a working channel of an endoscope. In another embodiment, the second jaw 612 may be formed with a base and body rotatable about a pivot point as with the first jaw 610. In yet another embodiment, both the first and second jaws 610, 612 may be formed with a solid construction and formed of an elastic material as disclosed above. The second jaw 612 also includes an opening 620 extending therethrough to slidably receive a cross-bar 630 therethrough, as will be described in greater detail below. A lower surface of the second jaw 612 includes a rail 617 having an angled protrusion 619 formed to lockingly engage the notch 628 in an operative configuration.

The cross-bar 630 extends from a distal end 632 permanently attached to the first jaw 610 to a proximal end (not shown) accessible to a physician or other user. In one embodiment, the proximal end (not shown) may be coupled to an actuation mechanism controlling movement of the cross-bar 630 through the endoscope (not shown). It is noted that although the cross-bar 630 is depicted with a rectangular cross-section, other cross-sectional shapes are envisioned within the scope of the disclosure including, but not limited to, circular, oval, oblong, square, etc. The cross-bar 630 includes a break point 634 formed as a reduced diameter portion thereof. In an insertion configuration, the break point 634 is seated between the first and second jaws 610, 612, as shown in FIG. 6. In a deployed configuration, the break point 634 is positioned proximally of the second jaw 612 so that, when the cross-bar 630 is broken at the break point 634, clip 606 is deployed in the body.

In an operative configuration, the clip 606 is inserted into a working channel of an endoscope (not shown). Walls of the working channel compress the first and second jaws 610, 612 to a reduced profile. This configuration bypasses the need for a larger diameter endoscope while still providing a clips jaws 610, 612 having a length sufficient to ensure that target tissue region is captured therebetween. The clip is advanced out of the endoscope to a desired position. When properly positioned, the control bar 630 is retracted proximally so the first jaw 610 moves proximally toward the second jaw 610. As the first jaw moves proximally, the rail 617 slides below the first jaw such that the angled protrusion 619 lockingly engages the notch 628. The endoscope (not shown) is then rotated or angled to cause the cross-bar 630 to fracture at the break point 634. Thus, a portion of the cross-bar 630 located distally of the break point 634 remains coupled to the clip 606 while a proximal portion thereof is withdrawn from the body through the endoscope.

FIG. 7 depicts a device 700 according to yet another embodiment, the device 700 being formed substantially similar to the device 600, wherein like elements are referenced with like reference numerals, except as noted below. Whereas the cross-bar 630 of the device 600 is permanently attached to the first jaw 610 at a distal end 632, the cross-bar 730 of the device 700 is removably attached to a first jaw 710. Specifically, the cross-bar 730 is connected to the first jaw 710 via a friction fit mechanism although other temporary attachment mechanisms are envisioned within the scope of the disclosure. The first and second jaws 710, 712 further including openings 114 extending therethrough to slidably receive the control wire 104 therethrough.

In an operative configuration, as clip 706 is slid out of an endoscope 102, the first and second jaws 710, 712 expand to a biased expanded configuration. The device is positioned in a target position relative to target tissue and the control wire 104 is retracted proximally to cause a corresponding proximal retraction of the first jaw 710. The first jaw 710 moves proximally until the angled protrusion 619 lockingly engages the notch 628. Further proximal retraction of the control wire 104 causes the abutment 128 to disengage the opening 114. The cross-bar 730 is then retracted proximally to cause a disengagement thereof from the first jaw 710. The cross-bar 730 is then retracted until the distal end 732 thereof is seated within an opening (not shown) extending through a second clip 706 positioned in the endoscope 102. It is noted that although the method is described with a separate disengagement of the control wire and cross-bar 730 from the first jaw 710, an alternate method may be directed to the simultaneous disengagement thereof. Specifically, once the clip 706 has been moved to the locked configuration, the control wire 104 and cross-bar 730 may be simultaneously retracted proximally to cause a simultaneous disengagement thereof from the clip 706. The exemplary device 700 permits the deployment of multiple clips 706 in a single procedure.

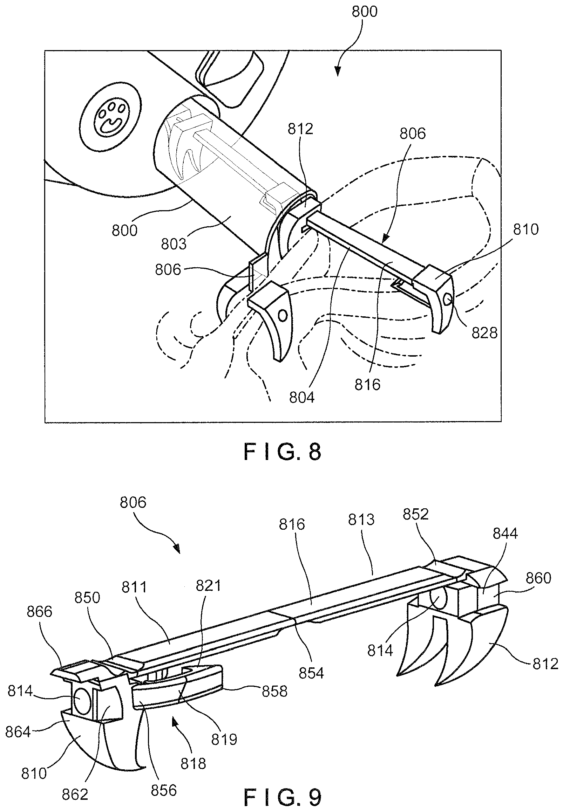

In another embodiment, as shown in FIGS. 8 - 15, a clipping device 800 is substantially similar to the clipping device 100 - 700 described above except as noted below. As described above in regard to the device 100, one or more clips 806 may be passed through an insertion member 802 for treating, for example, a tissue opening. The clip 806 comprises a first jaw 810 and a second jaw 812 coupled to each other via a collapsible member 816. The collapsible member 816 is movable between a biased open configuration, in which the collapsible member 816 extends along a substantially straight line, and a closed configuration, in which the collapsible member 816 bends at three bending points 850, 852, 854 to draw the first and second jaws 810, 812 toward one another to grip a tissue therebetween. Each of the first and second jaws 810, 812 includes an axially aligned opening 814 for receiving a control wire 804, substantially as described above in regard to the device 100.

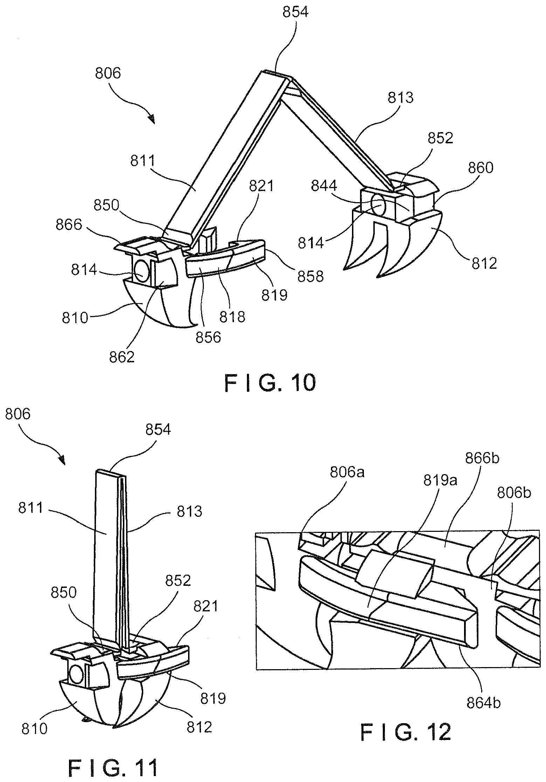

As shown in FIGS. 9 - 11, the collapsible member 816 includes at least three bending points - the first bending point 850 proximate the first jaw 810, a second bending point 852 proximate the second jaw 812 and a third bending point 854 at a joint 809 between first and second portions 811, 813 of the collapsible member. Thus, when collapsible member 816 is moved from the open position toward the closed position, the collapsible member 816 is bent at each of these bending points 850, 852, 854, moving the first and second jaws 810, 812 toward one another to grip tissue therebetween. As described above in regard to the clipping device 100, an angle at the third bending point 854 between the first and second portions 811, 813 of the collapsible member 816 may be approximately 180 degrees in the open configuration. When the collapsible member 816 is moved to the closed configuration, however, the angle between the first and second portions 811, 813 approaches 0 degrees, as shown in FIG. 11. While the angle at the bending point 854 may approach 0 degrees when the clip 106 is moved toward the closed position, the angles at the first and second bending points 850, 852 may approach 90 degrees. The collapsible member 816 along these bending points 850, 852, 854 may be thinned, for example, to approximately half the width of a remaining portion of the collapsible member 816. The thinned sections act as a hinge, permitting the collapsible member 816 to bend at these points. The bending points 850, 852, 854 may also be thinned to promote bending of the collapsible member 816 in a particular direction.

The clip 806, however, comprises a locking mechanism 818 including a pair of arms 819 extending from the first jaw 810 toward the second jaw 812, on opposing sides of the opening 814 extending therethrough. Each of the arms 819 extends from a first end 856 connected to a lateral surface of the first jaw 810 to a second end 858 extending proximally toward the second jaw 812. The second end 858 includes a tab 821 extending radially inward to engage a corresponding notch 844 of the second jaw 812. The second jaw 812 includes corresponding receiving notches 844 extending along lateral sides thereof so that, when the clip 806 is moved from the open configuration to the closed configuration, the tab 821 of each of the arms 819 engages a corresponding one of the notches 844. The receiving notches 844 extend longitudinally along lateral surfaces 845 thereof. A position of the receiving notches 844 along the lateral surfaces corresponds to a position of the arms 819 so that, when the collapsible member 816 is closed with the first and second jaws 810, 812 adjacent to one another, the tab 821 of each of the arms 819 engages a corresponding one of the receiving notches 844. The receiving notches 844 may include a chamfered surface 860 so that, when the tab 821 slides therealong, the pair of arms 819 engages the chamfered surface to be deflected radially outwardly. Once the tab 821 has passed proximally beyond the chamfered surface 860, a biasing force causes each of the arms 819 to deflect radially inward toward its original configuration. In this position, the tab 821 of each of the arms 819 engages a proximal wall of the second jaw 812, locking the first and second jaws 810, 812 in the closed configuration.

The first jaw 810 may also include a pair of distal notches 862 in a distal face 864 thereof, on opposing sides of the opening 814 so that when the locking mechanism 818 is moved toward the closed configuration, the tabs 821 of the arms 819 are received within the distal notches 862 of an immediately proximal clip 806, as shown in FIG. 12. The distal notches 862 are thus sized and shaped to permit arms 819 of an adjacent clip to be received therein, in the biased radially inward position. The distal face 864 may also include an overhang 866 extending distally therefrom to maintain adjacent clips 806 in alignment with one another and prevent rotation of a distal-most clip 806 about the control wire 804. In particular arms 819 of a distal-most clip 806a in the closed position is engaged via an overhang 866b of an immediately proximal clip 806b and received in distal notches 864b of the immediately proximal clip 806b to maintain an alignment therebetween.

Although the clip 806 is described as having arms 819 extending proximally from the first jaw 810 to engage receiving notches 844 of the second jaw 812, in another embodiment, arms may similarly extend distally from the second jaw 812 to engage receiving notches formed in the first jaw 810. It will be understood that distal notches would not be required in this embodiment.

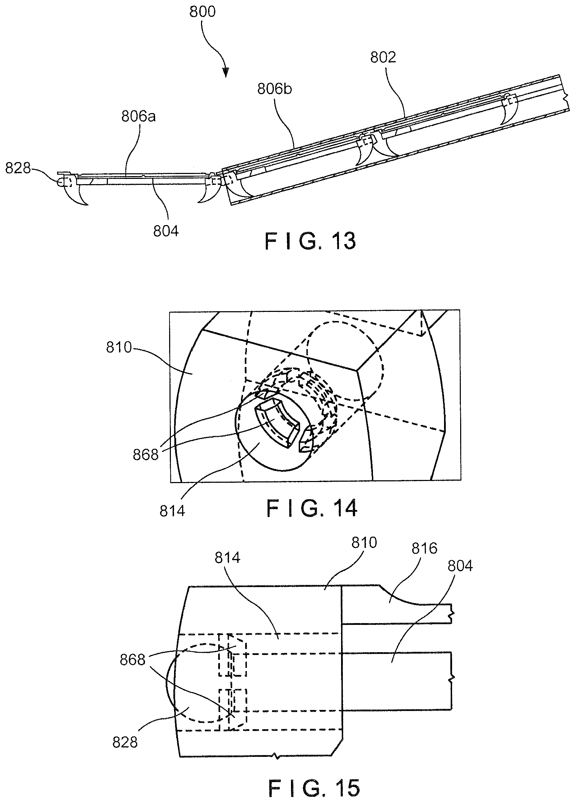

The clip 806 may be deployed from the insertion member 802 in a manner substantially similar to the device 100. In another embodiment, however, the control wire 804 includes a single enlarged portion 828 at a distal end thereof. The enlarged portion 828 engages the opening 814 of the first jaw 810 of a distal-most clip 806. The control wire 804 may also include a bend along a portion of a length thereof positioned along the control wire 804 so that, when the distal-most clip 806a is moved distally beyond a distal end 803 of the insertion member 802, the distal-most clip 806 is slightly angled with respect to the immediately proximal-clip 806b, as shown in FIG. 13. Thus, even when the insertion member 802 is inserted into a living body at an angle relative to a tissue opening to be treated, the distal-most clip 806a may be placed to move parallel to a surface of the tissue. Since the distal-most clip 806 is permitted to move parallel to the tissue surface, the first and second jaws 810, 812 may more properly grasp the opposing sides of the tissue wound.

As shown in FIGS. 14 - 15, the opening 814 of the first jaw 810 includes panels 868 extending radially thereinto to for engaging the enlarged portion 828 of the control wire 804. The panels 868 are designed to flex when a predetermined threshold force is exerted thereon via the enlarged portion 828. In this embodiment, the enlarged portion 828 is positioned distally of the panels 868. The panels 868 have a rigidity selected to be sufficient to support the first jaw 810 as a plurality of clips 806 housed within the insertion lumen 802 are moved distally relative thereto to move the distal-most clip 806a distally beyond the distal end 803 of the insertion member 802. Once the distal-most clip 806a has been positioned over a tissue opening to be treated (e.g., with the first jaw 810 along a first edge of the tissue opening and the second jaw 812 along a second edge of the tissue opening substantially opposing the first edge), the control wire 804 may be drawn proximally draw the first jaw 810 toward the second jaw 812 toward the closed configuration. The predetermined threshold force required to flex the panels 868 is selected to be greater than a force required to draw the clip 806 to the closed configuration. Once the jaws 810, 812 have been locked via the locking mechanism 818 as described above, the control wire 804 may be moved further proximally relative to the clips 806 until the panels 868 give way to the proximal force exerted thereon via the enlarged portion 828, releasing the enlarged portion 828 and permitting the distal-most clip 806a to be deployed. The control wire 804 is moved proximally until the enlarged portion engages the panels 868 of the immediately proximal clip 806b.

In another embodiment, rather than flexing to permit the enlarged portion 828 to be passed therebetween, releasing the enlarged portion 828 from the distal-most clip 806a, the panels 868 may be designed to snap off or crush under a predetermined threshold force. Substantially similarly to the embodiment described above, this predetermined threshold force is selected to be greater than the force required to move the first jaw 810 toward the second jaw 812 and lock the clip 806 in the closed configuration. Once the distal-most clip 806a has been locked in the closed configuration, the control wire 804 may be moved further proximally, exerting an additional proximal force on the panels 868 until the panels 868 are broken or crushed, deploying the distal-most clip 806a and permitting the enlarged portion 828 to engage the panels 868 of the immediately proximal clip 806b.

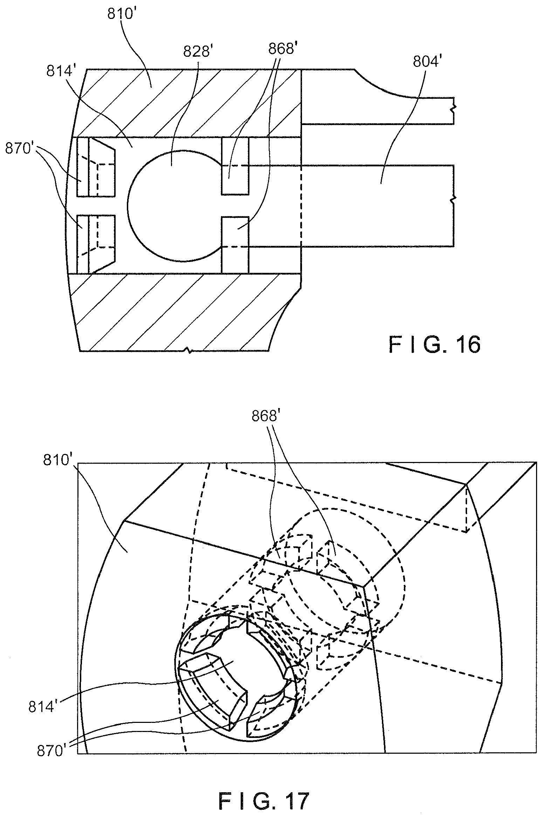

In a further embodiment, as shown in FIGS. 16 - 17, a first jaw 810' may include a first set of panels 868' and a second set of panels 870' extending radially into an opening 814' thereof. The first set of panels 868' may be positioned proximally of the second set of panels 870' so that an enlarged portion 828' of a control wire 804' is positionable therebetween. Substantially similarly to the clip 806 described above, the first set of panels 868' may be configured to break, snap off or be crushed under a first predetermined threshold pressure. This first predetermined threshold pressure is selected to be greater than the pressure required to close and lock the clip 806'. The second set of panels 870' may be configured to flex under a second predetermined threshold force. This second predetermined threshold force permits the enlarged portion 828' to be passed proximally therethrough to be housed between the first and second sets of panels 868', 870' while preventing the enlarged portion 828' from moving distally therepast while moving a distal-most clip 806' distally past a distal end of an insertion member.

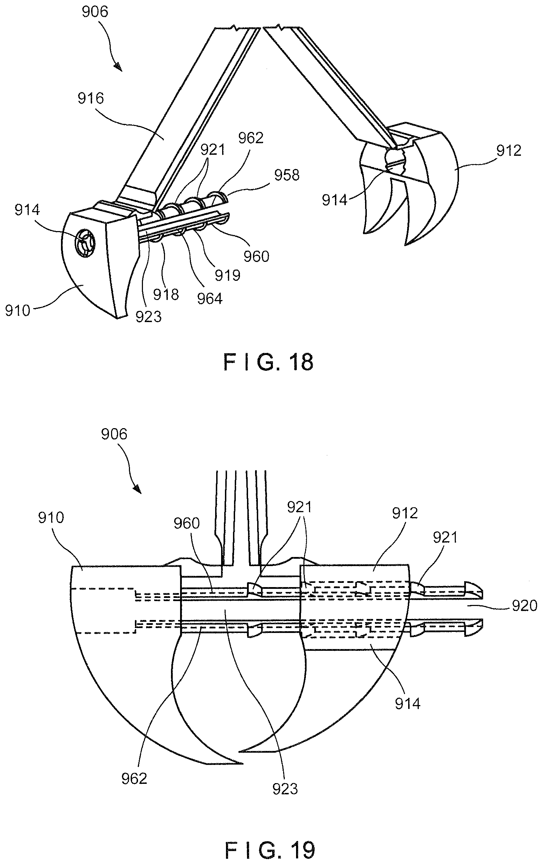

As shown in FIGS. 18 - 19, a clip 906 according to another exemplary embodiment of the present disclosure is also substantially similar to the clips 106 - 806 described above except as noted below. The clip 906 comprises a first jaw 910 and a second jaw 912 connected to another and movable relative to one another between an open configuration and a closed configuration via a collapsible member 916. A locking mechanism 918 that locks the first and second jaws 918 in the closed configuration, however, includes a tubular rod 919 extending proximally from a proximal face of the first jaw 910, a channel 920 extending longitudinally through the rod 919 in axial alignment with openings 914 extending through the first and second jaws 910, 912. Thus, when the clip 906 is moved toward the closed configuration, the tubular rod 919 is received within the opening 914 of the second jaw 912. The channel 920 is sized to slidably accommodate a control wire (e.g., control wire 104 or 804 from either of the devices 100, 800 described above) therein.

The tubular rod 919 includes a pair of longitudinal slots 923 extending therealong and diametrically opposing one another separating the tubular rod 919 into first and second portions 960, 962 that are deflectable toward one another to be received through the opening 914 of the second jaw 912. An exterior surface 964 of the tubular rod 919 may include a ridge 921 protruding therefrom to engage a proximal wall of the second jaw 912 upon being passed proximally through the opening 914 thereof. In another exemplary embodiment, the tubular rod 919 includes a plurality of ridges 921, which may be evenly spaced relative to one another, to allow different levels of closure of the clip 806, depending one the amount of tissue gripped between the first and second jaws 910, 912. The ridges 921 may be shaped so that the tubular rod 919 is slidable through the opening 914 of the second jaw 912 in a proximal direction but, once one of the ridges 921 is moved proximally therebeyond to engage a proximal wall of the second jaw 912, the tubular member 919 is prevented from moving distally relative to the second jaw 912. The opening 914 extending through the second jaw 912 are specifically sized and shaped to accommodate the tubular rod 919. For example, the opening 914 may include a pair of diametrically opposed grooves extending therealong for receiving the ridges first and second portions 960, 962 along with the control wire.

According to an exemplary method using the clip 906, once the clip 906 is located so that a desired portion of tissue is positioned between the first and second jaws 910, 912, the clip 906 is moved to the closed configuration via the control wire. As the first and second jaws 910, 912 are moved toward one another, a proximal end 958 of the tubular rod 919 enters the opening 914 of the second jaw 912, the first and second portions 960, 962 portions deflecting radially inward as the ridges 921 are received within the opening 914. Once a desired one of the ridges 921 extends proximally past the opening 914, the first and second portions 960, 962 revert to their original biased configuration relative to one another so that the desired one of the ridges extends proximally beyond the opening 914 to engage the proximal wall of the second jaw 912. Although the tubular rod 919 is shown and described as extending proximally from the first jaw 910 to be received within the second jaw 912, it will be understood by those of skill in the art that the tubular rod 919 may similarly extend distally from the second jaw 912 to be distally received within an opening of the first jaw 910.

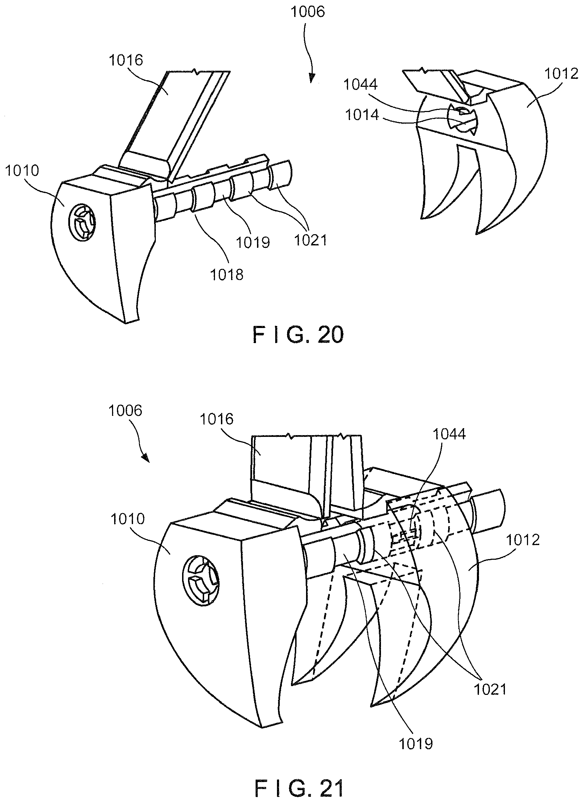

As shown in FIGS. 20 - 21, a clip 1006 according to another exemplary embodiment of the present disclosure is substantially similar to the clip 906 described above except as described below. The clip 1006 comprises a first jaw 1010 and a second 1012 connected to one another via a collapsible member 1016 for moving the clip 1006 between an open and a closed configuration. A locking mechanism 1018 of the clip 1006 includes a tubular rod 1019 substantially similar to the tubular rod 919 described in regard to the clip 906. Rather than ridges engaging a proximal wall of the second jaw 1012, however, the locking mechanism 1018 further includes a flexible member 1044 extending inward from a surface of the opening 1014. The flexible member 1044 is biased radially inward but is deformable as protrusions 1021 of the tubular member 1019 are moved proximally therepast. The protrusions 1021 and the flexible member 1014 are configured to permit the tubular rod 1019 to be moved proximally through the opening 1014, but prevent the tubular member 1019 from being slid distally therethrough. Thus, once a desired protrusion has moved proximally past the flexible member 1044, the first and second jaws 1010, 1012 are locked relative to one another.

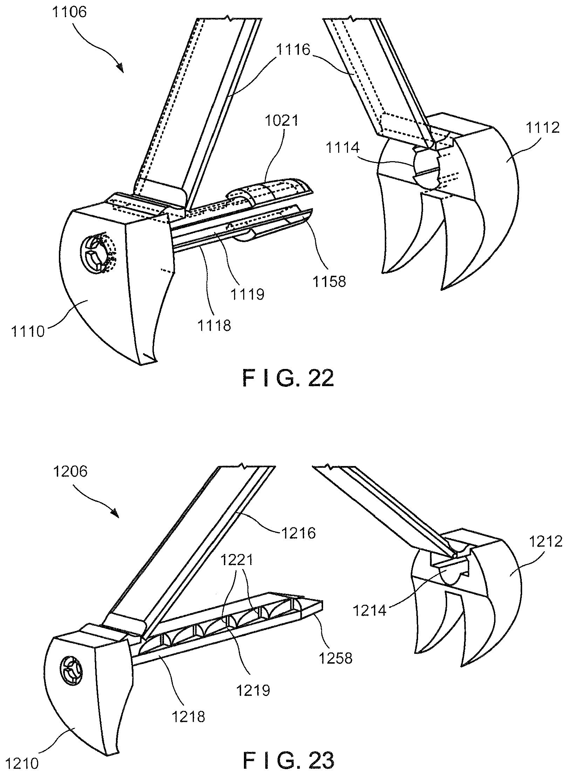

As shown in FIG. 22, a clip 1106 according to another exemplary embodiment of the present disclosure may be substantially similar to the clips 906, 1006 described above. Similarly to the clips 906, 1006, a locking mechanism 1118 of the clip 1106 includes a tubular rod 1119 extending proximally from a first jaw 1110 toward the second jaw 1112 to be received within an opening 1114 of the second jaw 1112. Rather than multiple ridges or protrusions extending along a length of the tubular rod 1119, however, the tubular rod 1119 includes an enlarged portion 1021 at a proximal end 1158 thereof. The enlarged end 1121 may extend from the proximal end 1158 along a length longer than either of the ridge 921 or protrusion 1021, which may provide a stronger hold between the tubular member 1119 and the second jaw 1112.

As shown in FIG. 23, a clip 1206 according to another exemplary embodiment of the present disclosure is substantially similar to the clips 906-1106 described above except as noted below. A locking mechanism 1218 of the clip 1206, however, includes a rectangular locking rod 1219 rather than a tubular rod. The locking rod 1219 extends proximally from a first jaw 1210 toward a second jaw 1212 to be received within an opening 1214 of the second jaw 1214. The locking rod 1219 extends substantially adjacent and parallel to a control wire received within the openings 1214 extending through first and second jaws 1210, 1212 so that, when the clip 1206 is moved to the closed configuration, a proximal end 1258 of the rod 1219 is received within the opening 1214 of the second jaw 1212. The opening 1214 of the second jaw 1212 is specifically sized and shaped to receive both the locking rod 1219 and the control wire. Since the locking rod 1219 is rectangularly shaped, once the locking rod 1219 is received within the opening 1214 of the second jaw 1212, the clip 1206 is prevented from rotating about the control wire.

The locking rod 1219 includes a plurality of notches 1021 along a length thereof. Similarly to the clip 1006, the opening 1214 of the second jaw 1212 includes a flexible member biased radially inward to engage the notches 1221 as the locking rod 1219 is slid proximally through the opening 1214. The notches 1221 are shaped (e.g., angled) so that the locking rod 1219 is permitted to be slid proximally through the opening 1214, but prevented from being moved distally therethrough.

Although the locking rods 919 - 1219 of the clips 906 - 1206 are described and shown as extending proximally from the first jaws 910 - 1210 to be received within openings 914 - 1214 of second jaws 912 - 1212, it will be understood by those of skill in the art that the locking rods 919 - 1219 may similarly extend distally from the second jaws 912 - 1212 to be received within openings of the first jaws 910 - 1210.

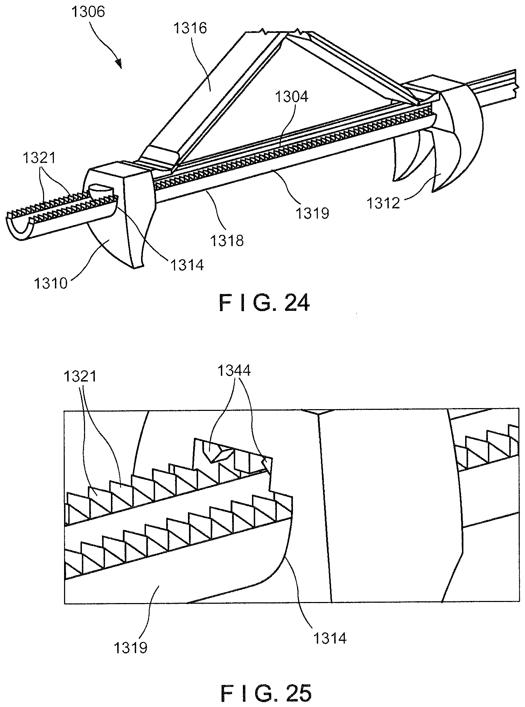

As shown in FIGS. 24 - 25, a clip 1306 according to another exemplary embodiment is substantially similar to the clips 906-1206 except as noted below. The clip 1306 comprises a first jaw 1310 and a second jaw 1312 connected to one another via a collapsible member 1316. A locking mechanism 1318, however, comprises a locking member 1319 extending distally from the second jaw 1312 to be received within an opening 1314 of the first jaw 1310. The lateral cross-section of the locking member 1319 may be substantially hemispherical to be received within the opening 1314 alongside a control wire 1304 received within the opening 1314. The locking member 1319 may extend along an underside of the control wire 1304 (e.g., a surface of the control wire 1304 facing a tissue to be treated in an operative position), to protect the control wire from tissue interference. The locking member 1319 includes ratchet teeth 1321 along longitudinal edges thereof for engaging a corresponding engaging element 1044 within the opening 1314. The ratchet teeth 1321 permit the locking member 1319 to be moved distally through the opening 1314 while preventing the locking member 1319 from being moved proximally therein.

The control wire 1304 in this embodiment is not required to be cylindrical. The control wire 1304 may, for example, be substantially rectangular with a rounded underside for engaging the hemispherical shape of the locking member 1319. The control wire 1304, however, may take any of a variety of shapes and sizes so long as the opening 1314 and the locking member 1319 are correspondingly sized and shaped.

Although the locking member 1319 is shown and described as extending distally from the second jaw 1312 to be received within the opening 1314 of the first jaw 1310, it will be understood by those of skill in the art that the locking member 1319 may similarly extend proximally from the first jaw 1310 to be proximally received within the opening of the second jaw 1312.

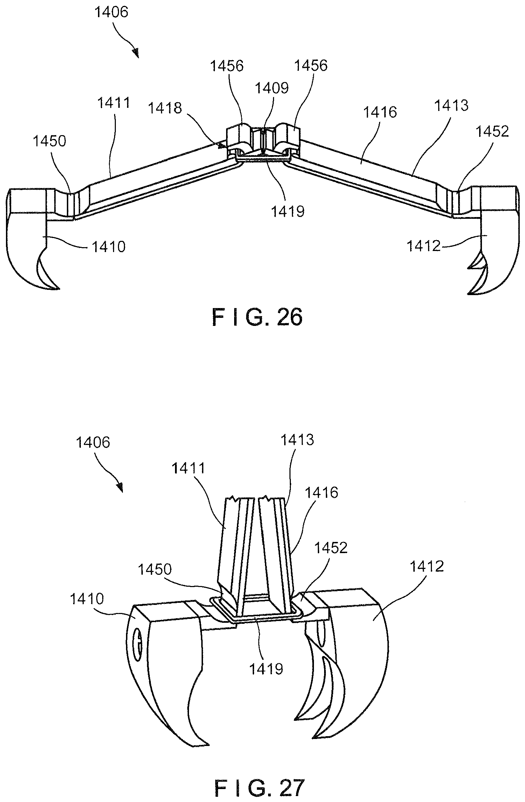

As shown in FIGS. 26 - 27, a clip 1406 is substantially similar to the clips described above except as noted below. The clip 1406 comprises a first jaw 1410 and a second jaw 1412 connected to one another via a collapsible member 1416 for moving the clip 1406 between open and closed configurations. A locking mechanism 1418 of the clip 1406, however, includes a ring 1419 movable over a length of the first and second portions 1411, 1413 of the collapsible member 1416 as the collapsible member 1416 is moved toward the closed configuration. In particular, the ring 1019 may slide from a joint 1409 connecting the first and second portions 1411, 1413 toward the first and second jaws 1410, 1412. In a locked closed configuration, the ring 1419 engages a thinned out portion of first and second bending points 1450, 1452 proximate the first and second jaws 1410, 1412, respectively. Once the ring 1419 have engaged the thinned out portions of the first and second bending points 1450, 1452, the strip is prevented from moving along the first and second portions 1411, 1413 toward the joint 1409. In the open configuration, the ring 1419 may engage hooks 1456 at the joint 1409, which prevent the ring 1419 from being disengaged from the collapsible member 1416, when the collapsible member 1416 is in a substantially straight configuration.

In one embodiment, the ring 1419 moves along the first and second portions 1411, 1413 toward the locked configuration via, e.g., the pull of gravity. Where the natural force of gravity is not utilized, the ring 1419 may include a feature that simulates the force of gravity. For example, a line under tension may be attached to the ring 1419 at two or more points, e.g., opposing sides of the ring 1419, attaching to one of the first and second jaws 1410. In one example, one or more lines are attached to the first jaw 1410, while one or more lines are attached to the second jaw 1412. Thus, as the first and second jaws 1410, 1412 are moved toward one another, the lines under tension draw the ring 1419 along the length of the first and second portions 1411, 1413 of the collapsible member 1419 toward the first and second jaws 1410, 1412 and into the thinned out portions of the first and second bending points 1450, 1452.

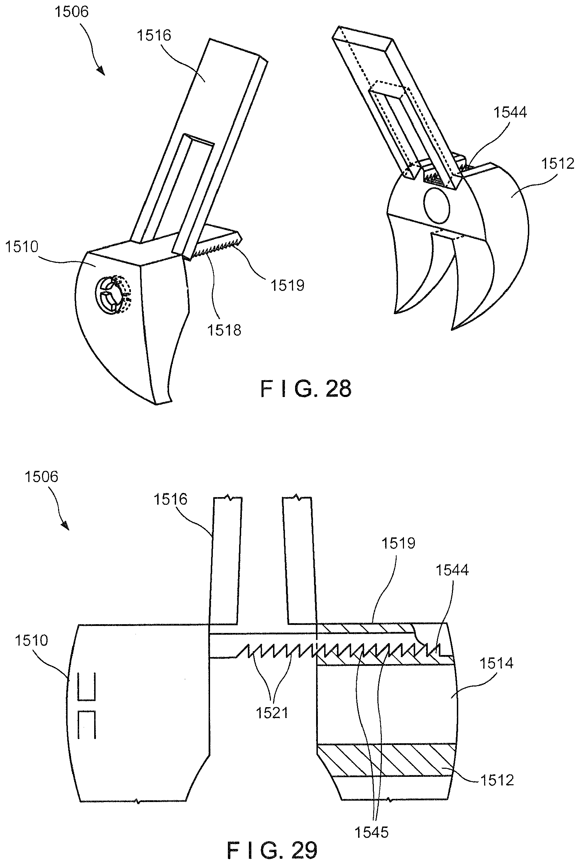

As shown in FIGS. 28 - 29, a clip 1506 according to another exemplary embodiment is substantially similar to the clips described above except as noted below. The clip 1506 comprises a first jaw 1510 and a second jaw 1512 connected to one another via a collapsible member 1516 for moving the clip 1506 between an open configuration and a closed configuration. A locking mechanism 1518 of the clip 1506, however, includes a latch 1519 extending proximally from the first jaw 1518 to be engagable with a corresponding engaging surface 1544 along the second jaw 1512. The latch 1519 include a plurality of ratchet teeth 1521 extending therealong while the engaging surface 1544 includes corresponding set of ratchet teeth 1545. The latch 1519 extends from a proximal face of the first jaw 1510 so that, when the clip 1506 is in the open configuration, the latch 1519 is substantially flush with the collapsible member 1516. When the clip 1506 is moved to the closed configuration, the latch 1519 passes through an opening 1560 in the collapsible member 1516 to engage the engaging surface 1544 of the second jaw 1512. The ratchet teeth 1521 and the corresponding ratchet teeth 1545 are configured so that the latch 1519 is permitted slide proximally relative to the engaging surface 1544 but prevented from moving distally relative thereto.

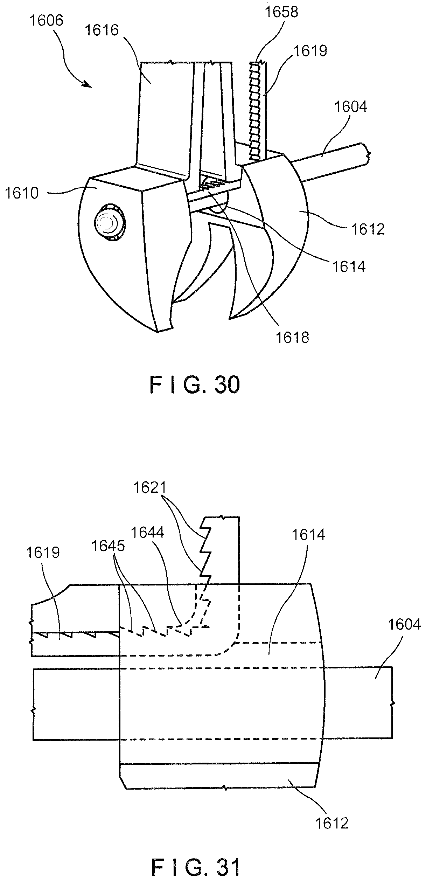

As shown in FIGS. 30 - 31, a clip 1606 according to another exemplary embodiment is substantially similar to the clips described above except as noted below. The clip 1506 comprises a first jaw 1610 and a second jaw 1612 connected to one another via a collapsible member 1616 for moving the clip 1606 between an open configuration and a closed configuration. A locking mechanism 1618 of the clip 1606, however, includes two flexible ratchet strips 1619 extending proximally from the first jaw 1610 adjacent and parallel to the collapsible member 1616, in the open configuration. The flexible ratchet strips 1619 may be substantially similar to zip-tie strips including ratchet teeth 1621 extending along lengths thereof and are received within an opening extending through the second jaw 1612. An interior surface of the opening 1614 includes corresponding ratchet teeth 1645 for engaging the ratchet teeth 1621. The second jaw 1612 also includes a pair of lateral openings 1644 extending therethrough in communication with the opening 1614 through which proximal ends 1658 of the ratchet strips 1619 extend out of.

In the open configuration, a length of the ratchet strips 1619 extend substantially parallel to the collapsible member 1616, with the proximal ends 1658 bent laterally to extend out of the lateral openings 1644. The proximal ends 1658 extend out of these lateral openings 1644 so that the ratchet strips 1619 do not interfere with an immediately proximal clip in an insertion member of a device (e.g., device 100, 800) through which this clip 1606 is inserted. As the clip 1606 is moved to the closed configuration, the proximal ends 1658 of the ratchet strips 1619 are drawn proximally so that ratchet teeth 1621 of the ratchet strips are slid proximally along the corresponding ratchet teeth 1654 of the opening 1614. The ratchet teeth 1621, 1654 are configured to permit the ratchet strips 1619 to be slid proximally through the opening 1614 but prevented from distally relative thereto.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The scope of the invention is, of course, defined in the language in which the appended claims are expressed.

## Claims

1. A clipping device (100), comprising:
a flexible insertion member (102) extending from a proximal end which, during use remains outside a body accessible to a user to a distal end which is inserted into the body through a naturally occurring body lumen;
a control wire (104) extending through the insertion member (102) from the proximal end; and
a first clip (106) including first and second jaws (110, 112), each of the first and second jaws (110, 112) including an opening (114) through which the control wire (104) passes and a collapsible member (116) coupled between the first and second jaws (110, 112), the collapsible member (116) being biased to urge the first and second jaws (110, 112) away from one another along an axis of the control wire (104) to a first expanded configuration, the first and second jaws (110, 112) being movable against the bias of the collapsible member (116) via the control wire (104) to a second contracted configuration in which the first and second jaws (110, 112) are moved along the axis of the control wire (104) toward one another to lockingly engage tissue received therebetween, **characterised in that** the flexible insertion member (102) defines a lumen (101) extending along an axial length thereof between the proximal end and the distal end, wherein the first clip (106) is housed within the lumen (101).

2. The clipping device (100) of claim 1, wherein the first clip (106) further comprises a first locking member (118) coupled to the first jaw (110) and extending from the first jaw (110) toward the second jaw (112), the first locking member (118) being constructed so that, when the first and second jaws (110, 112) are moved toward one another into the second configuration, the first locking member (118) lockingly engages the second jaw member (112).

3. The clipping device (100) of claim 2, wherein the first locking member (118) is formed as a rod (119) extending from the first jaw (110) toward the second jaw (112).

4. The clipping device (100) of claim 3, wherein a free end of the rod (119) of the first locking member (118) includes a locking arm (121) extending away from the rod (119) at an angle offset from a longitudinal axis of the rod (119) to engage the second jaw (112).

5. The clipping device (100) of claim 4, wherein a proximal surface of the free end of the locking arm (121) is angled relative to a surface of the second jaw (112) so that, as the free end of the rod (119) moves proximally into contact with the second jaw (112), the locking arm (121) deflects until the locking arm (121) passes over the second jaw (112) and locks against a proximal side thereof.

6. The clipping device (100) of claim 4, wherein the first jaw (110) extends from a first end including a first body engaging the collapsible member (116) to a second end including a first tissue gripping tip (122).

7. The clipping device (100) of claim 6, wherein the second jaw (112) extends from a first end including a second body engaging the collapsible member (116) to a second end including a second tissue gripping tip (124).

8. The clipping device (100) of claim 7, wherein the second end of the second jaw (112) includes a third tissue gripping tip (126), preferably the second and third tissue gripping tips (124, 126) being separated from one another by an opening (108), the opening (108) receiving at least a portion of the first jaw (110) therein in the second contracted configuration, the opening (108) includes an angled wall configured to lockingly engage the locking arm (121) of the first locking member (118).

9. The clipping device (100) of claim 7, wherein the control wire (104) includes an enlarged distal end (128) engaging a distal side of the opening (114) in the first jaw (110) so that proximal movement of the control wire (104) relative to the first jaw (110) draws the first jaw (110) proximally relative to the second jaw (112), the enlarged end (128) being configured to be movable to a second configuration to permit separation thereof from the first jaw (110) to release the first clip (106).

10. The clipping device (100) of claim 9, wherein the enlarged distal end (128) of the control wire (104) engages the opening (114) in the first jaw (110) with a pressure fit.

11. The clipping device (100) of claim 5, wherein the opening (914) in the second jaw (912) includes a rod receiving space and wherein the rod (919) is lockingly received within the opening (914) in the second contracted configuration.

12. The clipping device (100) of claim 1, wherein the collapsible member (116) includes first and second tabs (115, 117) received within corresponding slots formed in the first and second jaw (110, 112).

13. The clipping device (100) of claim 12, further comprising a collapsible body portion connecting the first and second tabs, including first and second portions (111, 113) connected to one another at a joint (109), wherein in the first expanded configuration, the first and second portions (111, 113) are moved apart to enclose a first angle (α) therebetween and wherein, in the second contracted configuration, the first and second portions (111, 113) enclose a second angle (α) smaller than the first angle, the second angle (α) preferably being 0.

14. The clipping device (100) of claim 1, wherein an outer profile of the first clip (106) is reduced when housed within the flexible insertion member (102), the outer profile expanding to a biased configuration when the first clip (106) is advanced out of the flexible insertion member (102).

15. The clipping device (100) of claim 1, further comprising a second clip (106) including third and fourth jaws (110, 112), each of the third and fourth jaws (110, 112) including an opening (114) through which the control wire (104) passes and a second clip collapsible member (116) coupled between the third and fourth jaws (110, 112), the second clip collapsible member (116) being biased to urge the third and fourth jaws (110, 112) away from one another along an axis of the control wire (104) to a first expanded configuration, the third and fourth jaws (110, 112) being movable against the bias of the second clip collapsible member (116) via the control wire (104) to a second contracted configuration in which the third and fourth jaws (110, 112) are moved along the axis of the control wire (104) toward one another to lockingly engage tissue received therebetween.

## Patentansprüche

1. Clipsetzvorrichtung (100), die aufweist:
ein flexibles Einführteil (102), das sich von einem proximalen Ende, das im Gebrauch außerhalb eines Körpers für einen Benutzer zugänglich bleibt, zu einem distalen Ende erstreckt, das in den Körper durch ein natürliches Körperlumen eingeführt wird;
einen Steuerdraht (104), der sich durch das Einführteil (102) vom proximalen Ende erstreckt; und
einen ersten Clip (106) mit einer ersten und einer zweiten Backe (110, 112), wobei die erste und zweite Backe (110, 112) jeweils eine Öffnung (114), die der Steuerdraht (104) durchläuft, und ein kollabierbares Teil (116) aufweisen, das zwischen der ersten und zweiten Backe (110, 112) gekoppelt ist, das kollabierbare Teil (116) vorgespannt ist, um die erste und zweite Backe (110, 112) entlang einer Achse des Steuerdrahts (104) in eine erste expandierte Konfiguration voneinander weg zu drücken, die erste und zweite Backe (110, 112) gegen die Vorspannung des kollabierbaren Teils (116) über den Steuerdraht (104) in eine zweite kontrahierte Konfiguration beweglich sind, in der die erste und zweite Backe (110, 112) entlang der Achse des Steuerdrahts (104) aufeinander zu bewegt sind, um dazwischen aufgenommenes Gewebe klemmend zu ergreifen,
**dadurch gekennzeichnet, dass** das flexible Einführteil (102) ein Lumen (101) definiert, das sich über eine Axiallänge davon zwischen dem proximalen Ende und dem distalen Ende erstreckt, wobei der erste Clip (106) im Lumen (101) untergebracht ist.

2. Clipsetzvorrichtung (100) nach Anspruch 1, wobei der erste Clip (106) ferner ein erstes Verriegelungsteil (118) aufweist, das mit der ersten Backe (110) gekoppelt ist und sich von der ersten Backe (110) zur zweiten Backe (112) erstreckt, wobei das erste Verriegelungsteil (118) so aufgebaut ist, dass bei Bewegung der ersten und zweiten Backe (110, 112) aufeinander zu in die zweite Konfiguration das erste Verriegelungsteil (118) mit der zweiten Backe (110) verriegelnd eingreift.

3. Clipsetzvorrichtung (100) nach Anspruch 2, wobei das erste Verriegelungsteil (118) als Stange (119) ausgebildet ist, die sich von der ersten Backe (110) zur zweiten Backe (112) erstreckt.

4. Clipsetzvorrichtung (100) nach Anspruch 3, wobei ein freies Ende der Stange (119) des ersten Verriegelungsteils (118) einen Verriegelungsarm (121) aufweist, der sich von der Stange (119) in einem gegenüber einer Längsachse der Stange (119) versetzten Winkel weg erstreckt, um einen Eingriff mit der zweiten Backe (112) herzustellen.

5. Clipsetzvorrichtung (100) nach Anspruch 4, wobei eine proximale Oberfläche des freien Endes des Verriegelungsarms (121) relativ zu einer Oberfläche der zweiten Backe (112) abgewinkelt ist, so dass bei proximaler Bewegung des freien Endes der Stange (119) in Kontakt mit der zweiten Backe (112) der Verriegelungsarm (121) abgelenkt wird, bis der Verriegelungsarm (121) die zweite Backe (112) überquert und gegen eine proximale Seite davon verriegelt.

6. Clipsetzvorrichtung (100) nach Anspruch 4, wobei sich die erste Backe (110) von einem ersten Ende mit einem ersten Körper, der einen Eingriff mit dem kollabierbaren Teil (116) herstellt, zu einem zweiten Ende mit einer ersten Gewebeerfassungsspitze (122) erstreckt.

7. Clipsetzvorrichtung (100) nach Anspruch 6, wobei sich die zweite Backe (112) von einem ersten Ende mit einem zweiten Körper, der einen Eingriff mit dem kollabierbaren Teil (116) herstellt, zu einem zweiten Ende mit einer zweiten Gewebeerfassungsspitze (124) erstreckt.

8. Clipsetzvorrichtung (100) nach Anspruch 7, wobei das zweite Ende der zweiten Backe (112) eine dritte Gewebeerfassungsspitze (126) aufweist, wobei vorzugsweise die zweite und dritte Gewebeerfassungsspitze (124, 126) durch eine Öffnung (108) voneinander getrennt sind, die Öffnung (108) mindestens einen Abschnitt der ersten Backe (110) darin in der zweiten kontrahierten Konfiguration aufnimmt, und die Öffnung (108) eine abgewinkelte Wand aufweist, die so konfiguriert ist, dass sie mit dem Verriegelungsarm (121) des ersten Verriegelungsteils (118) verriegelnd eingreift.

9. Clipsetzvorrichtung (100) nach Anspruch 7, wobei der Steuerdraht (104) ein vergrößertes distales Ende (128) aufweist, das einen Eingriff mit einer distalen Seite der Öffnung (114) in der ersten Backe (110) herstellt, so dass eine proximale Bewegung des Steuerdrahts (104) relativ zur ersten Backe (110) die erste Backe (110) relativ zur zweiten Backe (112) proximal zieht, wobei das vergrößerte Ende (128) so konfiguriert ist, dass es in eine zweite Konfiguration beweglich ist, um seine Trennung von der ersten Backe (110) zu ermöglichen, um den ersten Clip (106) freizusetzen.

10. Clipsetzvorrichtung (100) nach Anspruch 9, wobei das vergrößerte distale Ende (128) des Steuerdrahts (104) einen Eingriff mit der Öffnung (114) in der ersten Backe (110) mit einem Presssitz herstellt.

11. Clipsetzvorrichtung (100) nach Anspruch 5, wobei die Öffnung (914) in der zweiten Backe (912) einen Stangenaufnahmeraum aufweist und wobei die Stange (919) in der Öffnung (914) in der zweiten kontrahierten Konfiguration verriegelnd aufgenommen ist.

12. Clipsetzvorrichtung (100) nach Anspruch 1, wobei das kollabierbare Teil (116) eine erste und eine zweite Lasche (115, 117) aufweist, die in entsprechenden Schlitzen aufgenommen sind, die in der ersten und zweiten Backe (110, 112) gebildet sind.

13. Clipsetzvorrichtung (100) nach Anspruch 12, die ferner einen kollabierbaren Körperabschnitt, der die erste und zweite Lasche verbindet, mit einem ersten und einem zweiten Abschnitt (111, 113) aufweist, die an einem Gelenk (109) miteinander verbunden sind, wobei in der ersten expandierten Konfiguration der erste und zweite Abschnitt (111, 113) auseinander bewegt sind, um einen ersten Winkel (a) dazwischen einzuschließen, und wobei in der zweiten kontrahierten Konfiguration der erste und zweite Abschnitt (111, 113) einen zweiten Winkel (a) einschließen, der kleiner als der erste Winkel ist, wobei der zweite Winkel (a) vorzugsweise 0 ist.

14. Clipsetzvorrichtung (100) nach Anspruch 1, wobei ein Außenprofil des ersten Clips (106) bei Unterbringung im flexiblen Einführteil (102) verkleinert ist, wobei das Außenprofil in eine vorgespannte Konfiguration expandiert, wenn der erste Clip (106) aus dem flexiblen Einführteil (102) vorgeschoben ist.

15. Clipsetzvorrichtung (100) nach Anspruch 1, die ferner einen zweiten Clip (106) mit einer dritten und einer vierten Backe (110, 112) aufweist, wobei die dritte und vierte Backe (110, 112) jeweils eine Öffnung (114), die der Steuerdraht (104) durchläuft, und ein zweites kollabierbares Clipteil (116) aufweisen, das zwischen der dritten und vierten Backe (110, 112) gekoppelt ist, das zweite kollabierbare Clipteil (116) vorgespannt ist, um die dritte und vierte Backe (110, 112) entlang einer Achse des Steuerdrahts (104) in eine erste expandierte Konfiguration voneinander weg zu drücken, die dritte und vierte Backe (110, 112) gegen die Vorspannung des zweiten kollabierbaren Clipteils (116) über den Steuerdraht (104) in eine zweite kontrahierte Konfiguration beweglich sind, in der die dritte und vierte Backe (110, 112) entlang der Achse des Steuerdrahts (104) aufeinander zu bewegt sind, um dazwischen aufgenommenes Gewebe klemmend zu ergreifen.

## Revendications

1. Dispositif d'attache (100) comprenant :
un élément d'insertion flexible (102) s'étendant à partir d'une extrémité proximale qui, pendant l'utilisation, reste à l'extérieur d'un corps accessible pour un utilisateur, jusqu'à une extrémité distale qui est insérée dans le corps par une lumière corporelle naturelle ;
un fil de commande (104) s'étendant à travers l'élément d'insertion (102) à partir de l'extrémité proximale ; et
une première attache (106) comprenant des première et deuxième mâchoires (110, 112), chacune des première et deuxième mâchoires (110, 112) comprenant une ouverture (114) à travers laquelle passe le fil de commande (104) et un élément repliable (116) couplé entre les première et deuxième mâchoires (110, 112), l'élément repliable (116) étant sollicité pour pousser les première et deuxième mâchoires (110, 112) à distance l'une de l'autre le long d'un axe du fil de commande (104) dans une première configuration expansée, les première et deuxième mâchoires (110, 112) étant mobiles contre la sollicitation de l'élément repliable (116) via le fil de commande (104) dans une seconde configuration contractée dans laquelle les première et deuxième mâchoires (110, 112) sont déplacées le long de l'axe du fil de commande (104) l'une vers l'autre pour mettre en prise, par verrouillage, le tissu reçu entre elles, **caractérisé en ce que** l'élément d'insertion flexible (102) définit une lumière (101) s'étendant le long de sa longueur axiale entre l'extrémité proximale et l'extrémité distale, dans lequel la première attache (106) est logée à l'intérieur de la lumière (101).

2. Dispositif d'attache (100) selon la revendication 1, dans lequel la première attache (106) comprend en outre un premier élément de verrouillage (118) couplé à la première mâchoire (110) et s'étendant à partir de la première mâchoire (110) vers la deuxième mâchoire (112), le premier élément de verrouillage (118) étant construit de sorte que, lorsque les première et deuxième mâchoires (110, 112) sont déplacées l'une vers l'autre dans la seconde configuration, le premier élément de verrouillage (118) met en prise, par verrouillage, le deuxième élément de mâchoire (112).

3. Dispositif d'attache (100) selon la revendication 2, dans lequel le premier élément de verrouillage (118) est formé comme une tige (119) qui s'étend de la première mâchoire (110) vers la deuxième mâchoire (112).

4. Dispositif d'attache (100) selon la revendication 3, dans lequel une extrémité libre de la tige (119) du premier élément de verrouillage (118) comprend un bras de verrouillage (121) s'étendant à distance de la tige (119) à un angle décalé par rapport à un axe longitudinal de la tige (119) pour mettre en prise la deuxième mâchoire (112).

5. Dispositif d'attache (100) selon la revendication 4, dans lequel une surface proximale de l'extrémité libre du bras de verrouillage (121) est coudée par rapport à une surface de la deuxième mâchoire (112) de sorte que, lorsque l'extrémité libre de la tige (119) se déplace de manière proximale en contact avec la deuxième mâchoire (112), le bras de verrouillage (121) dévie jusqu'à ce que le bras de verrouillage (121) passe sur la deuxième mâchoire (112) et se verrouille contre son côté proximal.

6. Dispositif d'attache (100) selon la revendication 4, dans lequel la première mâchoire (110) s'étend à partir d'une première extrémité comprenant un premier corps mettant en prise l'élément repliable (116) jusqu'à une seconde extrémité comprenant une première pointe de préhension de tissu (122).

7. Dispositif d'attache (100) selon la revendication 6, dans lequel la deuxième mâchoire (112) s'étend à partir d'une première extrémité comprenant un second corps mettant en prise l'élément repliable (116) jusqu'à une seconde extrémité comprenant une deuxième pointe de préhension de tissu (124).

8. Dispositif d'attache (100) selon la revendication 7, dans lequel la seconde extrémité de la deuxième mâchoire (112) comprend une troisième pointe de préhension de tissu (126), de préférence des deuxième et troisième pointes de préhension de tissu (124, 126) qui sont séparées l'une de l'autre par une ouverture (108), l'ouverture (108) recevant au moins une partie de la première mâchoire (110) à l'intérieur de cette dernière dans la seconde configuration contractée, l'ouverture (108) comprend une paroi coudée configurée pour mettre en prise, par verrouillage, le bras de verrouillage (121) du premier élément de verrouillage (118).

9. Dispositif d'attache (100) selon la revendication 7, dans lequel le fil de commande (104) comprend une extrémité distale (128) agrandie mettant en prise un côté distal de l'ouverture (114) dans la première mâchoire (110) de sorte que le mouvement proximal du fil de commande (104) par rapport à la première mâchoire (110) tire la première mâchoire (110) de manière proximale par rapport à la deuxième mâchoire (112), l'extrémité (128) agrandie étant configurée pour être mobile dans une seconde configuration pour permettre sa séparation de la première mâchoire (110) afin de libérer la première attache (106).

10. Dispositif d'attache (100) selon la revendication 9, dans lequel l'extrémité distale (128) agrandie du fil de commande (104) met en prise l'ouverture (114) dans la première mâchoire (110) avec un ajustement de pression.

11. Dispositif d'attache (100) selon la revendication 5, dans lequel l'ouverture (914) dans la deuxième mâchoire (912) comprend un espace de réception de tige et dans lequel la tige (919) est reçue, par verrouillage, à l'intérieur de l'ouverture (914) dans la seconde configuration contractée.

12. Dispositif d'attache (100) selon la revendication 1, dans lequel l'élément repliable (116) comprend des première et seconde languettes (115, 117) reçues à l'intérieur de fentes correspondantes formées dans les première et deuxième mâchoires (110, 112).

13. Dispositif d'attache (100) selon la revendication 12, comprenant en outre une partie de corps repliable raccordant les première et seconde languettes, comprenant des première et seconde parties (111, 113) raccordées entre elles au niveau d'un joint (109), dans lequel dans la première configuration expansée, les première et seconde parties (111, 113) sont éloignées pour enfermer un premier angle (α) entre elles et dans lequel, dans la seconde configuration contractée, les première et seconde parties (111, 113) enferment un second angle (α) inférieur au premier angle, le second angle (a) étant de préférence 0.

14. Dispositif d'attache (100) selon la revendication 1, dans lequel un profil externe de la première attache (106) est réduit lorsqu'il est logé à l'intérieur de l'élément d'insertion flexible (102), le profil externe subissant une expansion jusqu'à une configuration sollicitée lorsque la première attache (106) est avancée hors de l'élément d'insertion flexible (102).

15. Dispositif d'attache (100) selon la revendication 1, comprenant en outre une seconde attache (106) comprenant des troisième et quatrième mâchoires (110, 112), chacune des troisième et quatrième mâchoires (110, 112) comprenant une ouverture (114) à travers laquelle passe le fil de commande (104) et un second élément repliable d'attache (116) couplé entre les troisième et quatrième mâchoires (110, 112), le second élément repliable d'attache (116) étant sollicité pour pousser les troisième et quatrième mâchoires (110, 112) à distance l'une de l'autre le long d'un axe du fil de commande (104) jusqu'à une première configuration expansée, les troisième et quatrième mâchoires (110, 112) étant mobiles contre la sollicitation du second élément repliable d'attache (116) via le fil de commande (104) jusqu'à une seconde configuration contractée dans laquelle les troisième et quatrième mâchoires (110, 112) sont déplacées le long de l'axe du fil de commande (104) l'une vers l'autre pour mettre en prise, par verrouillage, le tissu reçu entre elles.
